# EUROPEAN PATENT SPECIFICATION

(11) **EP 3 064 171 B1**
(45) Date of publication and mention of the grant of the patent: **06.01.2021**
(21) Application number: 14857256.3
(22) Date of filing: 31.10.2014
(51) Int. Cl.: A61F 2/07

(54) **COATED STENT GRAFT**
BESCHICHTETER STENT-GRAFT
ENDOPROTHÈSE COUVERTE

(30) Priority: 31.10.2013 CN 201310530470
(43) Date of publication of application: 07.09.2016
(73) Proprietor: Shanghai Microport Endovascular Medtech Co., Ltd., Shanghai 201318 (CN)
(72) Inventor: LI, Zhonghua, Shanghai 201203 (CN); WANG, Liwen, Shanghai 201203 (CN); ZHU, Qing, Shanghai 201203 (CN); GAO, Yanbin, Shanghai 201203 (CN)
(74) Representative: Lavoix
(86) International application number: PCT/CN2014/089972
(87) International publication number: WO 2015/062532

(56) References cited:
- WO-A1-00/28922
- CN-A- 1 170 612
- US-A- 5 314 472
- US-A1- 2007 123 972
- US-A1- 2007 123 972
- US-A1- 2010 114 294
- US-A1- 2010 198 333

## Description

### TECHNICAL FIELD

The invention relates to a stent graft, which can be used for treating the abdominal aneurysm and the iliac aneurysm.

### BACKGROUND

At present, there are many kinds of stent grafts for treating the abdominal aneurysm and the iliac aneurysm on the market, and the various products have different advantages and disadvantages, and have different treatment points. The stent graft for treating the abdominal aneurysm and the iliac aneurysm should have the following characteristics: the stent graft should be adapted to a circuitously bent blood vessel, and should have comparatively good adhesion and roundness properties, a moderate support strength and the like.

At present, on the domestic and international markets, there are mainly the following performance indexes to evaluate this kind of stent grafts: ① tightness: evaluating the validity of the stent isolating the tumor cavity from the blood; ② anti-displacement: preventing the displacement of the stent after the placement of the stent; ③ release resistance: a proper release resistance being capable of smoothly releasing the stent and avoiding the displacement of the stent; ④ positioning accuracy: evaluating the degree of offset between the positioning before the release and the position of the stent graft after the release; ⑤ stent flexibility: evaluating the ability of the stent graft being adaptively bent in a bent blood vessel; ⑥ adaptation to a bent tumor neck; and ⑦ a smaller tumor neck length.

Multiple stent grafts are disclosed in the prior art. For example, the US patent document US5800456 discloses a spiral zigzag stent section woven by a steel stainless wire, but the distance between the adjacent spiral stent sections is zero, i.e., the lower apex of the first ring overlaps the upper apex of the second ring, which design makes the whole outline increased and the bending properties poor; the apexes of this stent section cannot completely form a cross section, and thus cannot form a stent capable of performing a complete adhesion; and the diameters and lengths of the stent described in this US patent document both change greatly before and after the compression, and this US patent document only gives descriptions on an isodiametric cylindrical stent.

The US patent document US2002/0065550A1 discloses a stent graft having a helical stent section sandwiched by a double-layered ePTFE membrane. This stent section forms a stent graft with a graft after being woven on a flat plate. The weaving production efficiency on the flat plate is low, and the angle between the two adjacent edges when the stent described in this US patent document is woven on the flat plate is zero, so the spiral stent section will increase the included angle in the later treatment, and if the later treatment is nonuniform, the fatigue and strength of the apexes will be influenced. Moreover, the filament end of the stent graft is not treated in any way, which may cause damages on the graft, and this US patent document only gives descriptions on an isodiametric cylindrical stent.

The US patent document US5019090A discloses a stent woven from one filament, wherein a stent section woven on a flat plate forms a cylindrical stent after being rotated. The weaving production efficiency on the flat plate is low, and the angle between the two adjacent edges when the stent of one kind is woven on the flat plate is zero, so the spiral stent section will increase the included angle in the later treatment, and if the later treatment is nonuniform, the fatigue and strength of the apexes will be influenced. Moreover, the filament end of the stent graft is not treated in any way, which may cause damages on the graft, and this US patent document only gives descriptions on an isodiametric cylindrical stent.

The US patent document US5876432A discloses a stent graft structure, which is a spiral stent structure woven from a metal filament, but a flexible connection manner is adopted between the spirals, and the material adopted by this flexible connection manner is a filament material satisfying biocompatibility, e.g., polyethylene, polypropylene, collagen protein, etc. This US patent document states a lot how to select to use the graft material and the connection material, but the filament end of the weaving filament is not treated in any way, which may cause damages on the graft, and this US patent document only gives descriptions on an isodiametric cylindrical stent.

U.S. Patent Pub. No. US2007123972A1 discloses a modular stent graft assembly for repairing a ruptured abdominal aorta aneurysm and having an aortic section tubular graft and an iliac section tubular graft. The aortic section graft has a proximal attachment stent thereon for suprarenal attachment of the assembly to the aorta. A proximal end portion of the iliac section graft underlies the distal end portion of the aortic graft and presses outwardly thereagainst forming a friction fit, at a telescoping region. The assembly can be selected from an inventory containing a set of delivery systems of four size aortic section grafts and a set of delivery systems of four size iliac section grafts, that together accommodate a large majority of aneurysm sizes, and delivery systems containing four standard sizes of occludes.

U.S. Patent Pub. No. US2010198333A1 discloses an endoluminal prosthesis which may include a tubular graft extending in a longitudinal direction, where the graft has an inner surface forming a lumen extending a length of the graft. An elongate member may be attached to the graft in a circumferentially and longitudinally extending manner such that the elongate member forms a series of longitudinally spaced apart turns, each turn extending substantially around a circumference of the graft. The elongate member may torsion the graft in at least the circumferential direction and cause the graft to form circumferentially and longitudinally extending folds in the portions of the graft disposed between longitudinally adjacent turns of the elongate member.

### SUMMARY OF THE INVENTION

The object of the invention lies in developing a stent graft, which has improved bending properties.

To be specific, the invention provides a stent graft, which comprises a stent body and a graft fixed on the stent body, the stent body being divided into a plurality of stent sections along a center axis of the stent graft, and each stent section extending around the center axis by one circle, wherein each stent section is formed by connecting a plurality of wave rods end to end in a wavy structure, characterized in that the stent body consists of a small diameter section, a transition section and a large diameter section along the center axis, and the transition section connects the small diameter section with the large diameter section, wherein a distance between adjacent stent sections in the transition section is larger than a distance between adjacent stent sections in the small diameter section, and is smaller than a distance between adjacent stent sections in the large diameter section and wherein
an edge height of each stent section in the transition section is larger than an edge height of each stent section in the small diameter section, and is smaller than an edge height of each stent section in the large diameter section.

Preferably, each of the diameters and edge heights of the stent sections in the transition section exhibit a gradient gradual increase.

Preferably, the distance between adjacent stent sections in the small diameter section is between 0.5 mm and 2 mm, the distance between adjacent stent sections in the transition section is between 1 mm and 3 mm and is larger than the distance between adjacent stent sections in the small diameter section, and the distance between adjacent stent sections in the large diameter section is between 2 mm and 4 mm and is larger than the distance between adjacent stent sections in the transition section.

Preferably, the edge height of each stent section in the small diameter section is between 4 mm and 8 mm, the edge height of each stent section in the transition section is between 7mm and 10 mm, and the edge height of each stent section in the large diameter section is between 8 mm and 13 mm.

Preferably, the stent sections at each end portion of the stent body partially overlap the adjacent stent sections along the center axis, so that the stent sections at each end portion of the stent body have a flush end surface structure.

Preferably, the stent body is woven from a filament, the filament having a filament end, wherein
a connection tube is fixed on the filament end; or
a developing point or developing tube is fixed on the filament end; or
the filament end is bent to form a circular shape after a grinding treatment; or
firstly the filament end is made to undergo a round head grinding and polishing treatment, then the filament end is wrapped with a polymer material, and finally a suture line is used to perform suturing and binding and perform fixation on a tube formed by the polymer material.

Preferably, all the stent sections in the stent graft are woven from one memory alloy filament, wherein each stent section spirally extends around the center axis, and the plurality of stent sections are connected with each other.

Preferably, a pitch between the respective stent sections of the stent graft is between 5 mm and 10 mm.

Preferably, the height of the first wave rod in two adjacent wave rods of each stent section of the stent graft along the center axis is between 4 mm and 6 mm, the height of the second wave rod in the two adjacent wave rods along the center axis is between 3.5 mm and 5.5 mm, and an included angle between the first wave rod and the second wave rod in the two adjacent wave rods is between 30 and 90 degrees.

Preferably, the stent sections in the transition section and the small diameter section are woven from one filament into a spiral stent section portion, and the stent sections in the large diameter section are woven from another filament into an individual stent section portion.

As compared with the prior art, the invention has the following advantages:
(1) The stent graft according to the invention can improve its bending properties by means of a parameter setting;
(2) Both of the proximal and distal ends of the stent graft according to the invention can have a flush end surface design, so the pull-out force can be increased; and the branch stent graft according to the invention can increase its cooperative length with the main stent graft, and can ensure its roundness and adhesion properties; and
(3) The spiral parameters of the stent sections are obtained by precise calculations and a large number of experiments, and the stent sections can be molded by weaving one time, so the production efficiency can be improved.

### BRIEF DESCRIPTION OF DRAWINGS

In order to illustrate the technical solutions of the embodiments of the invention more clearly, the figures to be used in the descriptions of the embodiments will be briefly introduced below. It is obvious that the figures in the descriptions below are only some specific embodiments recorded in the present application, rather than limitations of the scope of protection of the invention. Those skilled in the art certainly can also obtain some other embodiments and figures according to these embodiments and their figures of the invention without making inventive efforts.
Fig. 1 is a schematic diagram of the whole appearance of the stent graft of the invention;
Fig. 2 is a schematic diagram of the change of the parameters of the stent sections around the transition section;
Fig. 3 is a schematic diagram of the stent graft being provided at its proximal end or distal end with a blank graft;
Fig. 4 is a schematic diagram of a flush end surface structure of the proximal end or distal end of the stent graft of the invention;
Fig. 5 is a schematic diagram of the filament end being connected by a connection tube;
Fig. 6 is a schematic diagram of the developing point or developing tube being fixed to the filament end;
Fig. 7 is a schematic diagram of the filament end being bent to form a circular shape;
Fig. 8 is a schematic diagram of the filament end being made to undergo a special treatment;
Fig. 9 is a schematic diagram of the value of the edge height of the stent section of the invention; and
Fig. 10 is a schematic diagram of the stent graft with an individual stent section portion.

### DETAILED DESCRIPTION

In order to make those skilled in the art better understand the technical solutions in the present application, clear and complete descriptions of the technical solutions in the embodiments of the invention will be given below by taking the figures in the embodiments of the invention into consideration. It is obvious that the described embodiments are only parts of the embodiments, rather than all of the embodiments, of the present application. Based on the detailed embodiments of the present application described below, all the other embodiments obtained by those skilled in the art without making inventive efforts shall fall within the scope of the concept of the invention.

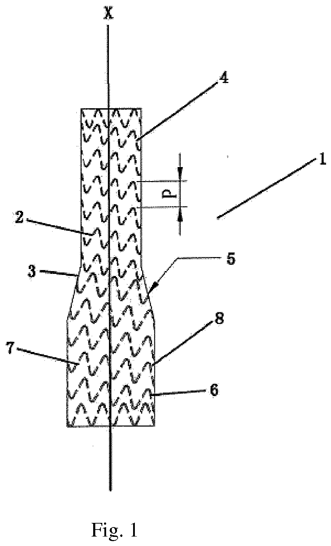

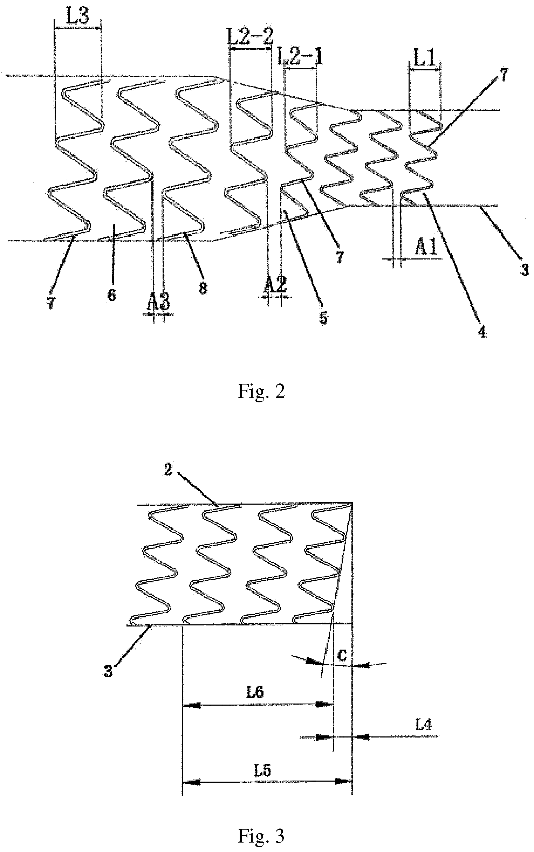

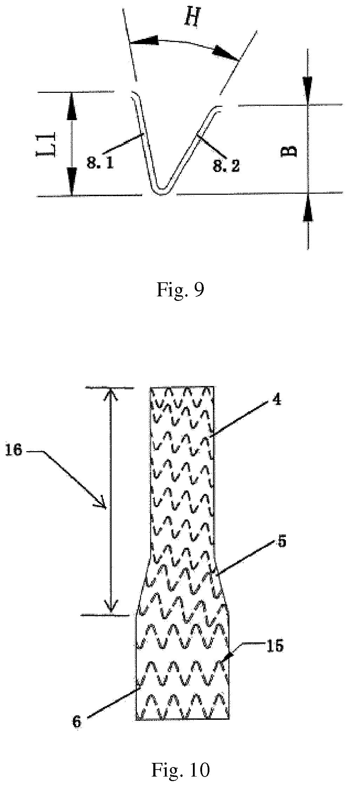

In the following descriptions, a proximal end refers to an end of the stent graft, after implantation in the human body, that is closer to the heart, and a distal end refers to an end of the stent graft, after implantation in the human body, that is farther from the heart. Specifically, in Figs. 1 and 10, the proximal end corresponds to an upper end and the distal end corresponds to a bottom end of the drawings; while in Figs. 2-4, the proximal end corresponds to a right end and the distal end corresponds to a left end of the drawings.

Fig. 1 is a schematic diagram of the whole appearance of the stent graft of the invention. As shown in Fig. 1, the stent graft 1 of the invention comprises a stent body 2 and a graft 3 fixed to the stent body 2. The stent body 2 is divided into a plurality of stent sections 7 along a center axis X of the stent graft 1, and each stent section 7 extends around the center axis X of the stent graft 1 by one circle. Each stent section 7 is formed by connecting a plurality of wave rods 8 end to end in a wavy structure. The stent body 2 consists of a small diameter section 4, a transition section 5 and a large diameter section 6 along the center axis X. The transition section 5 connects the small diameter section 4 with the large diameter section 6. The small diameter section 4 is smoothly transitioned to the large diameter section 6 via the transition section 5. The diameter of the large diameter section 6 is larger than the diameter of the small diameter section 4.

### 1. Change of the parameters of the filament at the place where the diameter changes

The invention adds a transition section 5 in the middle part of the stent graft having its proximal end and distal end changed in the diameter. With respect to the stent graft 1, assuming that the small diameter section 4 is directly connected to the large diameter section 6, the bending properties and other effects of the stent graft 1 are all comparatively poor, and especially a stent graft of a type that the diameter of the proximal end is greatly different from the diameter of the distal end needs a transition section 5 more.

The parameters of the stent sections of the transition section 5 are required to be changed correspondingly. In order to improve the flexibility of the whole stent graft, the key factors are a distance between the adjacent stent sections and an edge height of the stent section. Fig. 2 is a schematic diagram of the change of the parameters of the stent sections around the transition section.

As shown in Fig. 2, the distance between the adjacent stent sections in the transition section 5 is larger than the distance between the adjacent stent sections in the small diameter section 4, and is smaller than the distance between the adjacent stent sections in the large diameter section 6. That is, the distance between the adjacent stent sections is increased along with the increase of the diameter of the stent section. Those skilled in the art can understand that in the invention, the "distance between the adjacent stent sections" refers to a distance by which the adjacent stent sections are spaced apart from each other along the center axis X of the stent graft 1. As shown in Fig. 2, the distance A1 between the adjacent stent sections in the small diameter section 4 is between 0.5 mm and 2 mm, the distance A2 between the adjacent stent sections in the transition section 5 is between 1 mm and 3 mm, and the distance A3 between the adjacent stent sections in the large diameter section 6 is between 2 mm and 4 mm. If the distance between the adjacent stent sections in the small diameter section 4 is too large, the graft will be rendered severely folded; and if the distance between the adjacent stent sections in the small diameter section 4 is too small, the apexes of the stent sections will be made to overlap, which increases the whole profile and makes the bending properties of the stent become poor. If the distance between the adjacent stent sections in the large diameter section 6 is too small, the bending properties of the stent will be made to become poor, and the whole profile will be increased, which results in that the size of the required transport system becomes large.

Further, the edge height of each stent section 7 in the transition section 5 is larger than the edge height of each stent section 7 in the small diameter section 4, and is smaller than the edge height of each stent section 7 in the large diameter section 6. In the invention, the height of the wave rod 8 in each stent section 7 along the center axis X of the stent graft 1 is the edge height of this stent section. That is, along with the increase of the diameter of the stent section, the edge height of the stent section having the corresponding diameter will also increase. The edge height of the stent section having a large diameter is larger than the edge height of the stent section having a small diameter. As shown in Fig. 2, the edge height L1 of each stent section in the small diameter section 4 is between 4 mm and 8 mm, the edge height L2-1, L2-2 of each stent section in the transition section 5 is between 7mm and 10 mm, and the edge height L3 of each stent section in the large diameter section 6 is between 8 mm and 13 mm. At the transition section 5, along with the increase of the diameter of the horn mouth structure of the stent section, the edge height of the stent section will also change correspondingly. For example, in Fig. 2, the edge height L2-2 should be larger than the edge height L2-1. Sizes of the diameter and edge height of the stent sections 7 in the transition section 5 simultaneously exhibit a gradient gradual increase. That is, along with the gradual increase of the diameter of the stent sections 7 in the transition section 5, the edge height of the stent sections 7 in the transition section 5 also gradually increases. That is, the transition section 5 has a horn mouth structure (in Fig. 1 and Fig. 2, the transition section 5 has a small upper part and a large lower part), and the smaller the diameter of the stent section 7 in the transition section 5 is, the smaller the edge height thereof is. The edge height of the stent section should be reasonably set. If the edge height of the stent section is too large, the bending properties of the stent graft are comparatively poor; and if the edge height of the stent section is too small, the production process of the stent graft is comparatively difficult.

### 2. Flush end surfaces of the proximal and distal ends

If there are redundant grafts around the stent sections, after the compression, the grafts will be largely wrinkled, so the size of the stent graft will be severely compressed, so that the actual size of the stent graft is inconsistent with the nominal size, which may cause an improper selection of the size by the doctor, and which results in that the case that the selected stent does not match the blood vessel may occur.

If the structures of the both ends of the stent graft do not change, the spiral structure will continue from the proximal end to the distal end, so there will be a large number of blank grafts at the proximal end and the distal end, as shown in Fig. 3. Fig. 3 is a schematic diagram of the stent graft being provided at its proximal end or distal end with a blank graft.

If the stent sections at the proximal end are only woven according to the spiral stent sections, the apexes of the stent sections at the proximal end will extend along an oblique line on the whole in the side view, as shown in Fig. 3. In this case, an angle C will be formed between an end plane of the proximal end of the stent graft (the vertical plane on the right-most side in Fig. 3) and the oblique line. If the graft within the range of the angel C is kept, the case that the size of the stent graft is greatly reduced for the graft is greatly wrinkled after the assembly will be caused; and if the graft within the range of the angel C is not kept, the graft within the range of the angel C should be cut down, so a process is required to be added during the machining. Moreover, if the stent should be used together with other stent grafts, the cooperative overlapping length should be at least kept between 20 mm and 30 mm, and the cooperative length will be reduced if the redundant graft is cut down. As shown in Fig. 3, an actual cooperative length L5 is larger than an actual action length L6 by a length L4. This structure wastes the stent graft within the range of the length L4, and since the support strength and size of the stent graft within the range of the length L4 are greatly smaller than those of the remaining part having the stent sections, the pull-out force, the tightness, the adhesion and roundness properties and the other related physical properties of this part and the stent grafts used together with this part will be all reduced. A similar problem may also exist in the distal end of the stent graft.

In order to solve the above technical problem, the structures of the proximal and distal ends of the stent graft may be designed as flush end surface structures, which not only makes the proximal and distal ends be free of redundant grafts, but also can ensure that the overlapping length used together with the other stent grafts is sufficiently long, and the other properties such as the pull-out force, the support strength, the adhesion properties, the tightness and the roundness properties are also correspondingly improved.

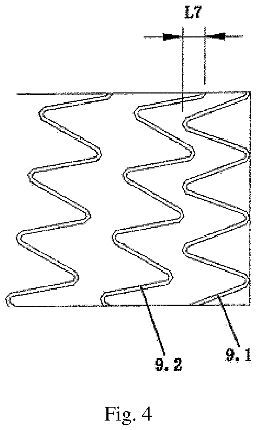

Fig. 4 is a schematic diagram of a flush end surface structure of the proximal end or distal end of the stent graft of the invention. As shown in Fig. 4, a stent section 9.1 at the proximal end of the stent body 2 of the invention partially overlaps an adjacent stent section 9.2 along the center axis X of the stent graft, so that the stent section 9.1 has a flush end surface structure. In this flush end surface structure, the proximal apexes (i.e. the apexes on the right side in the figure) of the flush end stent section 9.1 are located on the same plane that is substantially perpendicular to the center axis X of the stent graft and are flush with the end plane of the stent graft. In this case, an overlapping length L7 will exist between the distal apexes (i.e. the apexes on the left side in the figure) of the flush end stent section 9.1 and the corresponding proximal apexes of the adjacent stent section 9.2. The overlapping of the stent sections will result in the reduction of the bending properties of the stent. But, the stent section at the proximal end can be used together with the other stent grafts, and this part is located in the tumor cavity, so this part does not have a high requirement for the bending properties. Thus, this design does not influence the performance of the stent graft. It should be understood that the above-described flush end surface structure can be applied to both end portions of the stent graft of the invention. Preferably, the stent sections at the both end portions of the stent body of the stent graft partially overlap the adjacent stent sections along the center axis of the stent graft, so that the stent sections at the both end portions of the stent body both have a flush end surface structure. The structure of the large diameter section at the distal end of the stent graft is also located in the iliac aneurysm cavity, and only two to three apexes overlap, so the above design will not influence the bending properties of the stent graft.

### 3. Treatment of the filament end of the stent section

In the invention, the stent body 2 is woven from a filament, the filament having a filament end 10. If the filament end 10 of the stent section is not treated, the filament end 10 may make the graft worn, burst the graft or even hurt the blood vessel. But, since the diameter of the filament forming the stent body 2 is comparatively thin, even if the tip of the filament end 10 is ground to be comparatively smooth, the filament end 10 may still wear the graft. With respect to this problem, the invention provides the following several methods for protecting the filament end.

### 3.1 Protection of the filament end by a connection tube

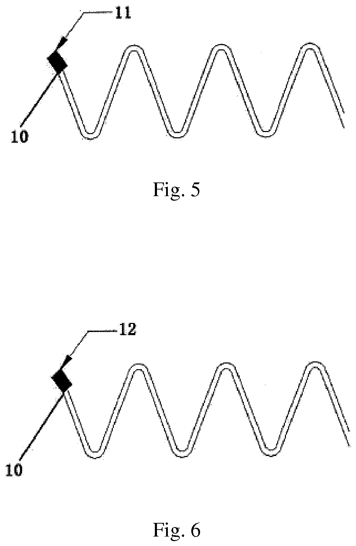

Fig. 5 is a schematic diagram of the filament end 10 being connected by a connection tube 11. Specifically, the stent body 2 of the stent graft 1 can be woven from one alloy filament as a whole, so the stent body 2 has only two filament ends 10 at both ends. In order to prevent the filament ends 10 from wearing the graft, a connection tube 11 may be fixed on the filament end 10. Specifically, the connection tube 11 is used to cover the filament end 10, so that the filament end 10 is hidden in the connection tube 11, and the connection pipe 11 and the alloy filament are connected by clamping by a plier or connected by soldering to thereby achieve the object of protecting the filament end 10.

### 3.2 Protection of the filament end by a developing point or developing tube

A developing point or developing tube is required to be sutured on the stent graft so that the position where the stent graft is located is clearly seen under an X-ray machine. The stent graft of the invention can use the developing point or developing tube in place of the above-described connection tube to clamp the filament end 10. Fig. 6 is a schematic diagram of the developing point (radiopaque) or developing tube 12 (radiopaque) being fixed on the filament end 10. In the manner shown in Fig. 6, not only the object of protecting the filament end 10 can be achieved, but also the purpose of developing can be served.

### 3.3. Bending treatment of the filament end

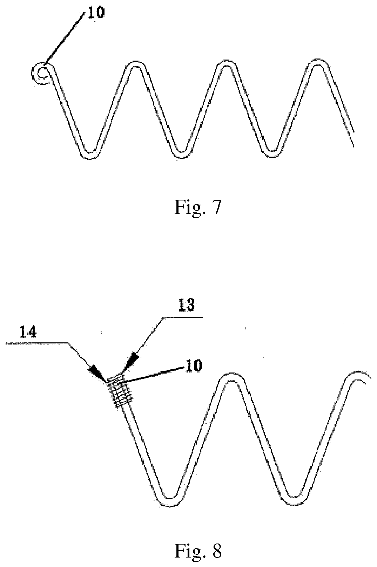

Fig. 7 is a schematic diagram of the filament end 10 being bent to form a circular shape. In the invention, the filament end 10 may be bent to form a circular shape after a grinding treatment to relieve the wear or damage of the filament end 10 on the graft.

### 3.4 Special treatment of the filament end

Fig. 8 is a schematic diagram of the filament end being made to undergo a special treatment. As shown in Fig. 8, firstly the filament end 10 may be made to undergo a round head grinding and polishing process, then the filament end 10 is wrapped with a polymer material 13 of a proper size, which polymer material may be a graft material, and finally a suture line 14 is used to perform suturing and binding and perform fixation on a tube formed by the polymer material 13 to prevent the filament end 10 from damaging or wearing the graft 3 fixed to the stent body 2.

### 4. Adoption of one filament to weave all of the stent sections

In the invention, all the stent sections 7 in the whole stent graft 1 may be woven from one memory alloy filament, wherein each stent section 7 spirally extends around the center axis X of the stent graft 1, and all of the stent sections 7 are connected with each other. Thus, the machining time can be reduced, and the connection of the connection tube is reduced to thereby reduce various corrosions between the metals. The stent grafts in the prior art may include a plurality of individual stent sections, wherein each stent section in the plurality of individual stent sections is connected by a connection tube. If the connection of the connection tube is improper, the stent section will be broken at the connection tube, so the filament end of the stent section may wear or damage the graft, and if the sizes of the connection tube and the individual stent section are not properly selected, a gap will be produced between the stent section formed of a metal filament and the connection tube formed of a metal tube to cause corrosion, which will result in the break of the stent section when severely.

The whole of the stent graft of the invention is woven from one filament. As shown in Fig. 1, a pitch P between the respective stent sections 7 of the stent graft 1 is between 5 mm and 10 mm. Different pitches may be designed according to the different diameters of the respective stent sections 7. If the pitch P is too small, the edge height of the stent section will be reduced, and although the small edge height of the stent section facilitates the bending, it does not facilitate the machining. If the pitch P is too large, the bending properties of the whole of the stent become poor.

The value of the pitch P depends on the values of the respective edge heights of the stent sections. Fig. 9 is a schematic diagram of the value of the edge height of the stent section of the invention. As shown in Fig. 9, the height L1 of the first wave rod 8.1 in the two adjacent wave rods of each stent section 7 of the stent graft 1 along the center axis X of the stent graft is between 4 mm and 6 mm, and the height B of the second wave rod 8.2 in the two adjacent wave rods along the center axis X is between 3.5 mm and 5.5 mm. If the lengths of the heights L1 and B are too short, weaving and stereotyping are not facilitated; and if the lengths of the heights L1 and B are too long, the bending properties of the stent are influenced. An included angle H between the first wave rod 8.1 and the second wave rod 8.2 is between 30 and 90 degrees. That the included angle H is too small and that the included angle H is too large will both influence the tensile strength and fatigue strength of the apex.

### 5. Separated structure

The stent graft as shown in Fig. 1 is an integrated structure having spiral stent sections. By considering that the support strength of the spiral stent section is slightly weak, an additional individual stent section portion 15 may be provided at the large diameter section 6 to improve the support strength of the stent graft 1. That is, the stent graft of the invention may also adopt a separated structure. Fig. 10 is a schematic diagram of the stent graft 1 with an individual stent section portion 15. As shown in Fig. 10, the stent sections 7 in the transition section 5 and the small diameter section 4 may be woven from one filament into a spiral stent section portion 16, and the stent sections 7 in the large diameter section 6 may be woven from another filament into an individual stent section portion 15. This individual stent section portion 15 may be a portion different from the spiral stent section, so the support strength of the stent graft 1 may be improved by this individual stent section portion 15.

The stent graft of the invention has improved bending properties, and has comparatively good roundness and adhesion properties, a moderate support strength and the like. Thus, the invention can be applied to the cases of various forms of circuitously bent abdominal and iliac aneurysms.

As compared with the prior art, the invention has the following advantages:
(1) The stent graft according to the invention can improve its bending properties by means of a parameter setting;
(2) Both of the proximal and distal ends of the stent graft according to the invention can have a flush end surface design, so the pull-out force can be increased; and the stent graft according to the invention can increase its overlapping length with the cooperative stent graft, and can ensure its roundness and adhesion properties; and
(3) The spiral parameters of the stent sections are obtained by precise calculations and a large number of experiments, and the stent sections can be molded by weaving one time, so the production efficiency can be improved.

The invention is mainly used for treating the abdominal and iliac aneurysms. During the operation, the stent graft is released after being guided to the lesion site, the metal stent supports the graft material on the normal blood vessel walls at both ends of the diseased blood vessel, so that the stent graft isolates the aneurysm from the blood flow within the blood vessel cavity to thereby eliminate the risk of break of the blood vessel and achieve the object of maintaining the blood flow of the aorta smooth; and the outer wall of the blood vessel is retracted due to the negative pressure, the normal blood vessel shape can be slowly restored, and the vascular intima cells permeate the micropores in the graft material and finally completely cover the stent graft to achieve endothelialization of the stent graft.

The stent graft of the invention can meet the various performance indexes to evaluate this kind of stent grafts as mentioned above in the Description, i.e., the stent graft has a very good tightness and thus can isolate the tumor cavity from the blood; the adoption of a proper release resistance can smoothly release the stent and avoid the displacement of the stent; the rear release function can be used to accurately position the stent graft; and the stent graft has a good flexibility. Thus, the invention can be adapted to various abdominal and iliac aortic aneurysms.

The above contents are only some detailed embodiments of the present application. It should be noted that those skilled in the art can also make various combinations or make some improvements and variations with respect to the above embodiments, and can also apply the invention to the treatment of other aneurysms in the case of not breaking away from the inventive principle and inventive concept of the present application, and these combinations, improvements, variations and applications should be also deemed as ones falling within the scope of protection and the inventive concept of the present application as long as covered by the claims language.

## Claims

1. A stent graft (1) comprising a stent body (2) and a graft (3) fixed on the stent body (2), the stent body (2) being divided into a plurality of stent sections (7) along a center axis (X) of the stent graft (1), each stent section (7) extending around the center axis (X) by one circle, wherein each stent section (7) is formed by connecting a plurality of wave rods (8) end to end in a wavy structure,
the stent body (2) consists of a small diameter section (4), a transition section (5) and a large diameter section (6) along the center axis (X), and the transition section (5) connects the small diameter section (4) with the large diameter section (6), **characterized in that**
a distance between adjacent stent sections in the transition section (5) is larger than a distance between adjacent stent sections in the small diameter section (4), and is smaller than a distance between adjacent stent sections in the large diameter section (6), and wherein
an edge height of each stent section (7) in the transition section (5) is larger than an edge height of each stent section (7) in the small diameter section (4), and is smaller than an edge height of each stent section (7) in the large diameter section (6).

2. The stent graft according to claim 1, **characterized in that**
each of the diameters and edge heights of the stent sections (7) in the transition section (5) exhibit a gradient gradual increase.

3. The stent graft according to claim 1, **characterized in that**
the distance (A1) between adjacent stent sections in the small diameter section (4) is between 0.5 mm and 2 mm, the distance (A2) between adjacent stent sections in the transition section (5) is between 1 mm and 3 mm and is larger than the distance (A1) between adjacent stent sections in the small diameter section (4), and the distance (A3) between adjacent stent sections in the large diameter section (6) is between 2 mm and 4 mm and is larger than the distance (A2) between adjacent stent sections in the transition section (5).

4. The stent graft according to claim 1, **characterized in that**
the edge height (L1) of each stent section in the small diameter section (4) is between 4 mm and 8 mm, the edge height (L2-1, L2-2) of each stent section in the transition section (5) is between 7 mm and 10 mm, and the edge height (L3) of each stent section in the large diameter section (6) is between 8 mm and 13 mm.

5. The stent graft according to any one of claims 1-4, **characterized in that**
the stent sections (9.1) at each end portion of the stent body (2) partially overlap adjacent stent sections (9.2) thereof along the center axis (X), so that the stent sections (9.1) at each end portion of the stent body (2) have a flush end surface structure.

6. The stent graft according to any one of claims 1-4, **characterized in that**
the stent body (2) is woven from a filament, the filament having a filament end (10), and a connection tube (11) is fixed on the filament end (10).

7. The stent graft according to any one of claims 1-4, **characterized in that**
the stent body (2) is woven from a filament, the filament having a filament end (10), and a developing point or developing tube (12) is fixed on the filament end (10).

8. The stent graft according to any one of claims 1-4, **characterized in that**
the stent body (2) is woven from a filament, the filament having a filament end (10), and the filament end (10) is bent to form a circular shape after a grinding treatment.

9. The stent graft according to any one of claims 1-4, **characterized in that**
the stent body (2) is woven from a filament, the filament having a filament end (10), and firstly the filament end (10) is made to undergo a round head grinding and polishing treatment, then the filament end (10) is wrapped with a polymer material (13), and finally a suture line (14) is used to perform suturing and binding and perform fixation on a tube formed by the polymer material (13).

10. The stent graft according to any one of claims 1-4, **characterized in that**
all the stent sections (7) in the stent graft (1) are woven from one memory alloy filament, wherein each stent section (7) spirally extends around the center axis (X), and the plurality of stent sections (7) are connected with each other.

11. The stent graft according to claim 10, **characterized in that**
a pitch (P) between the respective stent sections (7) of the stent graft (1) is between 5 mm and 10 mm.

12. The stent graft according to claim 10, **characterized in that**
the height (L1) of the first wave rod (8.1) in two adjacent wave rods of each stent section (7) of the stent graft (1) along the center axis (X) is between 4 mm and 6 mm, the height (B) of the second wave rod (8.2) in the two adjacent wave rods along the center axis (X) is between 3.5 mm and 5.5 mm, and an included angle (H) between the first wave rod (8.1) and the second wave rod (8.2) in the two adjacent wave rods is between 30 and 90 degrees.

13. The stent graft according to any one of claims 1-4, **characterized in that**
the stent sections (7) in the transition section (5) and the small diameter section (4) are woven from one filament into a spiral stent section portion (16), and the stent sections (7) in the large diameter section (6) are woven from another filament into an individual stent section portion (15).

## Patentansprüche

1. Stent-Graft (1), umfassend einen Stent-Körper (2) und einen Graft (3), der auf den Stent-Köper (2) fixiert ist, wobei der Stent-Körper (2) in eine Vielzahl von Stent-Abschnitte (7) entlang einer zentralen Achse (X) des Stent-Grafts (1) unterteilt ist, wobei sich jeder Stent-Abschnitt (7) um die zentrale Achse (X) um einen Kreis erstreckt,wobei jeder Stent-Abschnitt (7) durch Verbinden einer Vielzahl von Wellenstangen (8) Ende an Ende in einer Wellenstruktur gebildet ist,
der Stent-Körper (2) aus einem Abschnitt mit kleinem Durchmesser (4), einem Übertgangsabschnitt (5) und einem Abschnitt mit großem Durchmesser (6) entlang der zentralen Achse (X) besteht und der Übertragungsabschnitt (5) den Abschnitt mit kleinem Durchmesser (4) mit dem Abschnitt mit großem Durchmesser (6) verbindet, **dadurch gekennzeichnet, dass**
ein Abstand zwischen benachbarten Stent-Abschnitten im Übergangsabschnitt (5) größer als ein Abstand zwischen benachbarten Stent-Abschnitten im Abschnitt mit kleinem Durchmesser (4) und kleiner als ein Abstand zwischen benachbarten Stent-Abschnitten im Abschnitt mit großem Durchmesser (6) ist, und wobei
die Kantenhöhe jedes Stent-Abschnitts (7) im Übergangsabschnitt (5) größser als eine Kantenhöhe jedes Stent-Abschnitts (7) im Abschnitt mit kleinem Durchmesser (4) und kleiner als eine Kantenhöhe jedes Stent-Abschnitts (7) im Abschnitt mit großem Durchmesser (6) ist.

2. Stent-Graft nach Anspruch 1, **dadurch gekennzeichnet, dass**
jeder der Durchmesser und Kantenhöhen der Stent-Abschnitte (7) im Übergangsabschnitt (5) eine Erhöhung mit einem moderaten Gradienten aufweist.

3. Stent-Graft nach Anspruch 1, **dadurch gekennzeichnet, dass**
der Abstand (A1) zwischen benachbarten Stent-Abschnitten im Abschnitt mit kleinem Durchmesser (4) zwischen 0,5 mm und 2 liegt,der Abstand (A2) zwischen benachbarten Stent-Abschnitten im Übergangsabschnitt (5) zwischen 1 mm und 3 mm liegt und größer als der Abstand (A1) zwischen benachbarten Stent-Abschnitten im Abschnitt mit kleinem Durchmesser (4) ist und der Abstand (A3) zwischen benachbarten Stent-Abschnitten im Abschnitt mit großem Durchmesser (6) zwischen 2 mm und 4 mm liegt und größser als der Abstand (A2) zwischen benachbarten Stent-Abschnitten im Übergangsabschnitt (5) ist.

4. Stent-Graft nach Anspruch 1, **dadurch gekennzeichnet, dass**
die Kantenhöhe (L1) jedes Stent-Abschnitts im Abschnitt mit kleinem Durchmesser (4) zwischen 4 mm und 8 mm liegt, die Kantenhöhe (L2-1, L2-2) jedes Stent-Abschnitts im Übergangsabschnitt (5) zwischen 0,5 mm und 10 mm liegt und die Kantenhöhe (L3) jedes Stent-Abschnitts im Abschnitt mit großem Durchmesser (6) zwischen 8 mm und 13 mm liegt.

5. Stent-Graft nach einem der Ansprüche 1 - 4, **dadurch gekennzeichnet, dass**
die Stent-Abschnitte (9.1) an jedem Endabschnitt des Stent-Körpers (2) teilweise benachbarte Stent-Abschnitte (9.2) davon entlang der zentralen Achse (X) überlappen, so dass die Stent-Abschnitte (9.1) an jedem Endabschnitt des Stent-Körpers (2) eine bündige Endoberflächenstruktur aufweisen.

6. Stent-Graft nach einem der Ansprüche 1 - 4, **dadurch gekennzeichnet, dass**
der Stent-Körper (2) aus einem Faden gewebt ist, wobei der Faden ein Fadenende (10) aufweist und ein Verbindungsrohr (11), auf das Fadenende (10) fixiert ist.

7. Stent-Graft nach einem der Ansprüche 1 - 4, **dadurch gekennzeichnet, dass**
der Stent-Körper (2) aus einem Faden gewebt ist, wobei der Faden ein Fadenende (10) aufweist und ein Entwicklungspunkt oder Entwicklungsrohr (12) auf dem Fadenende (10) fixiert ist.

8. Stent-Graft nach einem der Ansprüche 1 bis 4, **dadurch gekennzeichnet, dass**
der Stent-Körper (2) aus einem Faden gewebt ist, wobei der Faden ein Fadenende (10) aufweist und das Fadenende (10) gebogen ist, um eine kreisförmige Ausgestaltung nach einer Mahlbehandlung zu bilden.

9. Stent-Graft nach einem der Ansprüche 1 - 4, **dadurch gekennzeichnet, dass**
der Stent-Körper (2) aus einem Faden gewebt ist, wobei der Faden ein Fadenende (10) aufweist und zunächst das Fadenende (10) hergestellt ist, um einem Rundkopfmahlen und einer Polierbehandlung unterzogen zu werden, dann das Fadenende (10) mit einem Polymermaterial (13) umwickelt wird und schließlich eine Nahtlinie (14) verwendet wird, um ein Nähen und Binden durchzuführen und eine Fixierung auf einem Rohr durchzuführen, das aus dem Polymermaterial (13) gebildet ist.

10. Stent-Graft nach einem der Ansprüche 1 - 4, **dadurch gekennzeichnet, dass**
alle Stent-Abschnitte (7) im Stent-Graft (1) aus einem Gedächtnislegierungsfaden gewebt sind, wobei sich jeder Stent-Abschnitt (7) spiralförmig um die zentrale Achse (X) erstreckt und die Vielzahl von Stent-Abschnitten (7) miteinander verbunden sind.

11. Stent-Graft (10) nach Anspruch 10, **dadurch gekennzeichnet, dass**
ein Zwischenraum (P) zwischen den entsprechenden Stent-Abschnitten (7) des Stent-Grafts (1) zwischen 5 mm und 10 mm liegt.

12. Stent-Graft (10) nach Anspruch 10, **dadurch gekennzeichnet, dass**
die Höhe (L1) der ersten Wellenstange (8.1) in zwei benachbarten Wellenstangen jedes Stent-Abschnitts (7) des Stent-Grafts (1) entlang der zentralen Achse (X) zwischen 4 mm und 6 mm liegt. die Höhe (13) der zweiten Wellenstange (8.2) in den zwei benachbarten Wellenstangen entlang der zentralen Achse (X) zwischen 3,5 mm und 5,5 mm liegt. und ein eingeschlossener Winkel (H) zwischen der ersten Wellenstange (8.1) und der zweiten Wellenstange (8.2) in den zwei benachbarten Wellenstangen zwischen 30 und 90 Grad liegt,

13. Stent-Graft nach einem der Ansprüche 1 - 4, **dadurch gekennzeichnet, dass**
die Stent-Abschnitte (7) im Übergangsabschnitt (5) und dem Abschnitt mit kleinem Durchmesser (4) aus einem Faden in einen spiralförmigen Stent-Abschnitteil (16) gewebt sind und die Stent-Abschnitte (7) im Abschnitt mit großem Durchmesser (6) aus einem anderen Faden in einen individuellen Stent-Abschnitteil (15) gewebt sind.

## Revendications

1. Endoprothèse (1) comprenant un corps de stent (2) et un greffon (3) fixé sur le corps de stent (2), le corps de stent (2) étant divisé en une pluralité de sections de stent (7) le long d'un axe central (X) de l'endoprothèse (1), chaque section de stent (7) s'étendant autour de l'axe central (X) sur un cercle, dans laquelle chaque section de stent (7) est formée en raccordant une pluralité de tiges ondulées (8) bout à bout dans une structure ondulée,
le corps de stent (2) se compose d'une section de petit diamètre (4), d'une section de transition (5) et d'une section de grand diamètre (6) le long de l'axe central (X), et la section de transition (5) raccorde la section de petit diamètre (4) avec la section de grand diamètre (6), **caractérisée en ce que** :
une distance entre les sections de stent adjacentes dans la section de transition (5) est supérieure à une distance entre les sections de stent adjacentes dans la section de petit diamètre (4) et est inférieure à une distance entre les sections de stent adjacentes dans la section de grand diamètre (6), et dans laquelle :
une hauteur de bord de chaque section de stent (7) dans la section de transition (5) est supérieure à une hauteur de bord de chaque section de stent (7) dans la section de petit diamètre (4), et est inférieure à une hauteur de bord de chaque section de stent (7) dans la section de grand diamètre (6).

2. Endoprothèse selon la revendication 1, **caractérisée en ce que** :
chacun des diamètres et chacune des hauteurs de bord des sections de stent (7) dans la section de transition (5) présentent une augmentation avec un gradient progressif.

3. Endoprothèse selon la revendication 1, **caractérisée en ce que** :
la distance (A1) entre les sections de stent adjacentes dans la section de petit diamètre (4) est comprise entre 0,5 mm et 2 mm, la distance (A2) entre les sections de stent adjacentes dans la section de transition (5) est comprise entre 1 mm et 3 mm et est supérieure à la distance (A1) entre les sections de stent adjacentes dans la section de petit diamètre (4) et la distance (A3) entre les sections de stent adjacentes dans la section de grand diamètre (6) est comprise entre 2 mm et 4 mm et est supérieure à la distance (A2) entre les sections de stent adjacentes dans la section de transition (5).

4. Endoprothèse selon la revendication 1, **caractérisée en ce que** :
la hauteur de bord (L1) de chaque section de stent dans la section de petit diamètre (4) est comprise entre 4 mm et 8 mm, la hauteur de bord (L2-1, L2-2) de chaque section de stent dans la section de transition (5) est comprise entre 7 mm et 10 mm, et la hauteur de bord (L3) de chaque section de stent dans la section de grand diamètre (6) est comprise entre 8 mm et 13 mm.

5. Endoprothèse selon l'une quelconque des revendications 1 à 4, **caractérisée en ce que** :
les sections de stent (9.1) au niveau de chaque partie d'extrémité du corps de stent (2) chevauchent partiellement sur ses sections de stent (9.2) adjacentes le long de l'axe central (X), de sorte que les sections de stent (9.1) au niveau de chaque partie d'extrémité du corps de stent (2) ont une structure de surface d'extrémité de niveau.

6. Endoprothèse selon l'une quelconque des revendications 1 à 4, **caractérisée en ce que** :
le corps de stent (2) est tissé à partir d'un filament, le filament ayant une extrémité de filament (10) et un tube de raccordement (11) est fixé sur l'extrémité de filament (10).

7. Endoprothèse selon l'une quelconque des revendications 1 à 4, **caractérisée en ce que** :
le corps de stent (2) est tissé à partir d'un filament, le filament ayant une extrémité de filament (10) et un point de développement ou tube de développement (12) est fixé sur l'extrémité de filament (10).

8. Endoprothèse selon l'une quelconque des revendications 1 à 4, **caractérisée en ce que** :
le corps de stent (2) est tissé à partir d'un filament, le filament ayant une extrémité de filament (10), et l'extrémité de filament (10) est pliée afin de former une forme circulaire après un traitement de meulage.

9. Endoprothèse selon l'une quelconque des revendications 1 à 4, **caractérisée en ce que** :
le corps de stent (2) est tissé à partir d'un filament, le filament ayant une extrémité de filament (10), et tout d'abord l'extrémité de filament (10) est réalisée pour subir un traitement de meulage et de polissage à tête ronde, ensuite l'extrémité de filament (10) est enveloppée avec un matériau polymère (13) et enfin une ligne de suture (14) est utilisée pour réaliser la suture et la liaison et réaliser la fixation sur un tube formé par le matériau polymère (13).

10. Endoprothèse selon l'une quelconque des revendications 1 à 4, **caractérisée en ce que** :
toutes les sections de stent (7) dans l'endoprothèse (1) sont tissées à partir d'un filament en alliage à mémoire de forme, dans laquelle chaque section de stent (7) s'étend, en spirale, autour de l'axe central (X), et la pluralité de sections de stent (7) sont raccordées entre elles.

11. Endoprothèse selon la revendication 10, **caractérisée en ce que** :
un pas (P) entre les sections de stent (7) respectives de l'endoprothèse (1) est compris entre 5 mm et 10 mm.

12. Endoprothèse selon la revendication 10, **caractérisée en ce que** :
la hauteur (L1) de la première tige ondulée (8.1) dans deux tiges ondulées adjacentes de chaque section de stent (7) de l'endoprothèse (1) le long de l'axe central (X) est comprise entre 4 mm et 6 mm, la hauteur (B) de la seconde tige ondulée (8.2) dans les deux tiges ondulées adjacentes le long de l'axe central (X) est comprise entre 3,5 mm et 5,5 mm, et un angle inclus (H) entre la première tige ondulée (8.1) et la seconde tige ondulée (8.2) dans les deux tiges ondulées adjacentes est compris entre 30 et 90 degrés.

13. Endoprothèse selon l'une quelconque des revendications 1 à 4, **caractérisée en ce que** :
les sections de stent (7) dans la section de transition (5) et la section de petit diamètre (4) sont tissées à partir d'un filament dans une partie de section de stent en spirale (16), et les sections de stent (7) dans la section de grand diamètre (6) sont tissées à partir d'un autre filament dans une partie de section de stent individuelle (15).
